# EUROPÄISCHE PATENTSCHRIFT

(11) **EP 4 076 264 B1**
(45) Veröffentlichungstag und Bekanntmachung des Hinweises auf die Patenterteilung: **26.07.2023**
(21) Anmeldenummer: 21782475.4
(22) Anmeldetag: 20.09.2021
(51) Int. Cl.: A61B 50/34, A61B 90/98, G16H 40/20, G16H 40/40, G16H 40/63, A61B 90/90, A61L 2/24, A61L 2/26, G06K 19/077

(54) **MODULARES ERFASSUNGSSYSTEM FÜR STERILKREISLAUFÜBERWACHUNG UND VERFAHREN ZUR ÜBERWACHUNG EINES STERILGUTKREISLAUFS**
MODULAR DETECTION SYSTEM FOR STERILE CIRCUIT MONITORING AND METHOD FOR MONITORING A STERILE MATERIAL CIRCUIT
SYSTÈME DE DÉTECTION MODULAIRE POUR SURVEILLANCE DE CIRCUIT STÉRILE ET PROCÉDÉ DE SURVEILLANCE D'UN CIRCUIT DE MATÉRIAU STÉRILE

(30) Priorität: 25.09.2020 DE 102020125114
(43) Veröffentlichungstag der Anmeldung: 26.10.2022
(73) Patentinhaber: Aesculap AG, 78532 Tuttlingen (DE)
(72) Erfinder: LENZENHUBER, Frederick, 78532 Tuttlingen (DE); BÖHLER, Lukas, 78120 Furtwangen (DE); MATEUSZ, Daniol, 32-241 Kozlów (PL); HÖGERLE, Roland-Alois, 78532 Tuttlingen (DE); AUBER, Stephanie, 78532 Tuttlingen (DE); PFISTER, Ralf, 78647 Trossingen (DE)
(74) Vertreter: Winter, Brandl - Partnerschaft mbB
(86) Internationale Anmeldenummer: PCT/EP2021/075773
(87) Internationale Veröffentlichungsnummer: WO 2022/063718

(56) Entgegenhaltungen:
- EP-A2- 2 416 298
- DE-A1-102015 108 264
- US-A1- 2007 094 303

## Beschreibung

### Technisches Gebiet

Die vorliegende Offenbarung betrifft ein modulares Erfassungssystem zum Einlegen in einen Siebkorb zum Erfassen, Überwachen und Rückverfolgen von Sterilgut, ein Halterungssystem, ein Siebkorbsystem sowie ein Verfahren zur Überwachung eines Sterilgutkreislaufs mit zumindest einem modularen Erfassungssystem.

### Hintergrund der Erfindung

Für die Überwachung und Rückverfolgbarkeit von Sterilgütern muss festgestellt werden können, ob medizintechnische/ chirurgische Instrumente und/ oder Geräte/ Devices und/ oder Motorensysteme und/ oder Medizinprodukte die gesamte Wiederaufbereitung erfolgreich durchlaufen haben und alle nötigen Wartungsschritte durchgeführt wurden. Hierbei entsteht das Problem, dass Sensoren nur in größeren Sterilgütern untergebracht werden können und daher insbesondere für medizintechnische/ chirurgische Instrumente keine Möglichkeit der Überwachung besteht. Ein weiteres Problem stellt die Verbindung von smarten Halterungssystemen, sprich von Halterungen mit einer Sensorelektronik, in einem nicht speziell dafür designten Siebkorb dar.

Halterungsvorrichtungen der vorliegenden Gattung werden unter anderem zum Reinigen und Halten von Motoren, Handstücken, chirurgischer Instrumente und generell von Medizinprodukten bereitgestellt. Die Halterungen werden mit in den Halterungen angeordneten Medizinprodukten in medizinische Reinigungsanlagen, insbesondere Sterilisatoren, Reinigungs- und Desinfektionsanlagen (RDG, Spülanlagen) oder Ähnlichen, angeordnet/ eingesetzt! verwendet. Darüber hinaus besteht auch die Möglichkeit, dass die Halterungen zusammen mit Sterilcontainern oder Weichverpackungen verwendet und innerhalb dieser angeordnet werden können, sodass nach einer Sterilisation der Medizinprodukte gewährleistet ist, dass eine neuerliche Kontamination der Medizinprodukte wirksam unterbunden ist.

### Stand der Technik

Der Stand der Technik beschreibt Halterungssysteme für medizinische Motorensystem, die mit Sensoren, einem Kommunikationsmodul und einer Datenverarbeitungseinheit ausgerüstet sind. Hierbei zeigt die US 2008/142 605 A1 eine Markierungsvorrichtung für einen Lager- und/oder Transportbehälter für medizinische Geräte mit einer Matte aus sterilisierbarem Material und einem Markierungselement zur Speicherung elektronischer Information über charakteristische Parameter der in der Lagerung gelagerten und/ oder transportierten medizinischen Geräte und Transportbehälter, welche drahtlos ausgelesen werden können, wobei ein RFID-Tag beispielsweise als Markierungselement verwendet wird, in dem Informationen über Sterilisationsprozesse, Sterilisationszeit und/ oder andere Parameter gespeichert sind, die für die im Lager- und/ oder Transportbehälter untergebrachten medizinischen Geräte charakteristisch sind.

Die DE 10 2011 050 333 A1 bezieht sich auf ein chirurgisches Behälterdetektionssystem, mit welchem der Inhalt eines Sterilisationsbehälters einfach und sicher bestimmbar ist, mit einer Behälterinhaltssensoreinrichtung zum Anordnen in oder an einem Sterilisationsbehälter, wobei die Behälterinhaltssensoreinrichtung einen Träger und mindestens einen am Träger angeordneten oder ausgebildeten Sensor zum Detektieren eines Identifizierungselements, welches an einem Sterilisationsbehälter gelagerten Gegenstand zu dessen Identifikation angeordnet oder ausgebildet ist, aufweist.

Die EP 2 416 298 A4 offenbart ein tragbares Behälterinventar-Kontrollsystem, das die RFID-Technologie zur automatischen Überwachung des Entnahme- und Rückgabevorgangs von Gegenständen, wie Werkzeugen, Waffen, Schmuck, chirurgischen Instrumenten aus einem oder mehreren Behältern in einem tragbaren Behälter, um den Status eines jeden Elements sowie eine Betriebsaufzeichnung eines jeden Elements aufrechtzuerhalten.

Die EP 2 914 195 B1 bezieht sich auf verschiedene Systeme und Verfahren zum Markieren und Verfolgen von chirurgischen Geräten/ Instrumenten unter Verwendung von RFID-Tags, welche die Verfolgung chirurgischer Geräte während ihrer Verteilung und Sterilisation ermöglichen. In einer Ausführungsform umfasst das System eine Schale, die konfiguriert ist, um mehrere chirurgische Vorrichtungen aufzunehmen, und an die ein übergeordnetes RFID-Tag angebracht ist, das Informationen enthält und/ oder den Zugang zu Informationen über die Schale und über jede der darinsitzenden chirurgischen Vorrichtungen erleichtert. An jedem der in der Instrumentenschale sitzenden chirurgischen Geräte kann ein untergeordneter RFID-Tag angebracht sein, der Informationen enthält und/ oder den Zugang zu Informationen über das chirurgische Gerät erleichtert.

Die WO 2009/003231 A1 beschreibt ein System zur Identifizierung von Gegenständen, die einer Sterilisation unterzogen werden, mit einem Behälter zum Halten von Gegenständen, die mit RFID-Etiketten versehen sind, die einen Sterilisationsprozess überstehen können. Der Behälter hat eine oder mehrere interne Antennen. Das System umfasst auch einen Container-Interrogator/Vernehmer zum Abfragen der Artikel-Tags über die internen Antennen des Containers, wobei der Interrogator/Vernehmer entfernbar mit dem Container gekoppelt ist.

Die DE 10 2015 108 264 A1 beschreibt ein chirurgisches bzw. medizinisches Behälterinhalt-Erfassungssystem mit einer Behälterinhalt-Sensoreinrichtung zum Anordnen in einem Sterilisationsbehälter, wobei die Behälterinhalt-Sensoreinrichtung einen Träger und mindestens einen am Träger angeordneten oder ausgebildeten Sensor zum Erfassen mindestens eines Identifizierungselements, welches an einem im Sterilisationsbehälter gelagerten Gegenstand zu dessen Identifikation angeordnet oder ausgebildet ist, umfasst. Ein an dem Träger angeordnetes Trägermodul weist eine Erfassungseinrichtung zur Erfassung mindestens eines erfassten Identifizierungselements auf und ist dazu angeordnet, Information über den durch das erfasste Identifizierungselement identifizierten Gegenstand drahtlos nach außerhalb des Sterilisationsbehälters zu übermitteln.

Die US 2007/094 303 A1 offenbart ein automatisiertes System zum Identifizieren, Analysieren und Aufzeichnen von Informationen im Zusammenhang mit Desinfektions- und/oder Sterilisationsverfahren.

Die bereits bekannten Halterungssysteme aus dem Stand der Technik haben mehrere Nachteile. Zum einen können die einzelnen Halterungssysteme nicht miteinander kommunizieren, jedes Halterungssystem benötigt eine komplette Sensor- und Kommunikationselektronik sowie eine eigenständige Antenne und zum anderen ist pro Halterungssystem je eine Control Unit/ Steuereinheit/ Elektronikeinheit notwendig.

### Kurzbeschreibung der Erfindung

Daher liegt der vorliegenden Offenbarung die Aufgabe zugrunde, ein System bereitzustellen, welches die Nachteile aus dem Stand der Technik behebt oder zumindest verbessert. Des Weiteren ist es eine Aufgabe der Erfindung, insbesondere bei einer hohen Stückzahl, die Herstellungskosten zu senken.

Diese Aufgabe wird durch ein modulares Erfassungssystem mit den Merkmalen des Patentanspruchs 1 bzw. durch ein Halterungssystem bzw. ein Siebkorbsystem mit zumindest einem solchem modularen Erfassungssystem bzw. mit einem Verfahren zur Überwachung eines Sterilgutkreislaufs mit zumindest einem solchen modularen Erfassungssystem gelöst. Vorteilhafte Ausgestaltungen und Weiterbildungen der Erfindung sind Gegenstand der Unteransprüche.

Die vorliegende Offenbarung betrifft demzufolge ein modulares Erfassungssystem zum Einlegen in einen Siebkorb zum Erfassen, Überwachen und Rückverfolgen von Sterilgut, insbesondere von chirurgischen Instrumenten und/oder Motorensystemen und/oder Medizinprodukten in einem Sterilisationskreislauf. Das modulare Erfassungssystem ist hierbei als ein Siebkorbeinsatz vorgesehen und ausgebildet, der direkt oder auf einer Basisplatte angeordnet in den Siebkorb einlegbar ist. Das modulare Erfassungssystem ist mit den folgenden individuell und wahlweise elektrisch koppelbaren Modulen vorgesehen und ausgebildet:
- einer zentralen Elektronikeinheit, die insbesondere mit zumindest einem Sensor, einer Energiespeichereinheit, einer Datenverarbeitungseinheit und einer Schreib- und Ausleseeinheit, vorzugsweise mittels NFC, ausgebildet ist,
- einer (definierten) Anzahl an Steckplätzen zum Anschließen und/ oder Verbinden einer (vorbestimmten) Anzahl an Halterungssystemen, die zum Halten von Sterilgut und mit jeweils zumindest einem Antennensystem zum Auslesen eines Transponders, vorzugsweise eines NFC-Transponders, des Sterilguts vorgesehen und ausgebildet ist, und
- einer (festgelegten) Anzahl an Leiterbahnen, insbesondere Daten- und Antennenleitungen und/oder -verbindungen, welche derart vorgesehen und ausgebildet sind, um die Steckplätze und/oder Halterungssysteme miteinander und/ oder mit der zentralen Elektronikeinheit elektrisch zu verbinden.

In anderen Worten betrifft die vorliegende Offenbarung ein modulares Sterilgut-Erfassungssystem, das zum Einlegen in einen Siebkorb für einen Sterilisationskreislauf vorbereitet ist, mit folgenden individuell und wahlweise elektrisch koppelbaren Modulen:
- einer zentralen Elektronikeinheit,
- einem elektrischen Leitungsaufbau, der an die Elektronikeinheit anschließbar ist und eine Anzahl von freien Anschlussterminals/ Steckplätzen hat, und
- eine Anzahl von, vorzugsweise unterschiedlich dimensionierten Sterilguthalterungen/ -auflagerungen zum individuellen Halten von Sterilgut, die jeweils mit zumindest einem zum freien Anschlussterminal kompatiblen Anschluss und wenigstens einer Antenneneinheit versehen sind, wobei die wenigstens eine Antenneneinheit mit dem zumindest einen Anschluss über eine Sterilisationshalterung/ -auflagerung-interne Leitung/ Leiterbahn elektrisch verbunden ist.

Im Allgemeinen sieht die vorliegende Offenbarung eine modulare Anbindung von mehreren Antennensystemen an eine zentrale Elektronikeinheit vor. Hierbei ist das gesamte Erfassungssystem so aufgebaut, dass es als ein Einsatz für einen Siebkorb fungiert und dort entweder direkt angebracht oder über einen entfernbaren Einsatz realisiert werden kann. Ein Vorteil eines solchen Einsatzes bzw. der Verwendung einer Basisplatte mit Daten- und Antennenleitungen und individuellen Halterungssystemen besteht darin, dass die Herstellung von speziell angepassten Leiterplatten nicht nötig ist.

In anderen Worten, weist das Modul der zentralen Elektronikeinheit/ Steuerungseinheit bevorzugt eine Energiequelle, vorzugsweise einen wiederaufladbaren Energiespeicher, eine Datenverarbeitungseinheit, ein Kommunikationsmodul, vorzugsweise ein Bluetooth oder WiFi-Kommunikationsmodul bzw. andere Funktechnologien, und einen RFID-Reader bzw. bevorzugterweise einen NFC-Reader, wahlweise mit Schreibfunktion, auf. Das Modul der Leiterbahnen, vorzugsweise Daten- und Antennenverbindungen, hat bevorzugt eine festgelegte Anzahl an Leiterbahnen, die mit der Datenverbindungseinheit und den AntennenSchnittstellen der zentralen Elektronikeinheit verbunden/ verbindbar sind. Die einzelnen Leiterbahnen enden in einer vorzugsweise festgelegten Anzahl an Kontaktstellen, die Steckplätze bilden, welche zur Aufnahmen von zumindest einem Halterungssystem vorgesehen sind. Unter dem Modul der individuellen Halterungssysteme sind Halterungen zu verstehen, welche an unterschiedliches Sterilgut, insbesondere Instrumente und/ oder Motorensysteme und/ oder Medizinprodukte, angepasst sind und über ein einfaches Sensorsystem vorzugsweise mit einem Energiespeicher und einer einfachen Datenverarbeitungseinheit verfügen. Weiterhin verfügt das zumindest eine Halterungssystem über zumindest eine Daten- und Antennenschnittstelle. Zum Auslesen von RFID bzw. bevorzugterweise NFC-Transpondern, welche in den einzelnen Sterilgütern vorgesehen sind, verfügt das zumindest eine Halterungssystem über ein Antennensystem, welches eine oder mehrere Antennen aufweist, die entweder direkt oder über einen (weiteren) Multiplexer mit der zentralen Elektronikeinheit verbunden sind.

Demnach ist es möglich, ein System bereitzustellen, dessen mehrere Antennensysteme mit einer einzigen/ genau einer zentralen Steuereinheit/ Elektronikeinheit verbunden werden können. Dadurch können individuelle Halterungs-/ Auflagerungssysteme mit einer minimierten Elektronik ausgerüstet werden. Unter einer minimierten Elektronik ist die Ausstattung der Halterungssysteme mit einfachen Sensoren, insbesondere Temperatur.-, Druck.-, Feuchtigkeits.-, Vibrations.-, Neigungs-, Bewegungs- und PH-wertsensoren und einer wiederaufladbaren Energiequelle/ Energiespeichereinheit zu verstehen. Dadurch können Prozessschritte, insbesondere Sterilisationsprozessschritte, Sterilisationszeiten, und andere Sterilgutkreislauf-Parameter erfasst und die Sensorwerte zwischengespeichert werden. Hierbei ist eine wenn möglich vollständige Überwachung des Sterilgutkreislaufs ein Ziel der vorliegenden Offenbarung. Demnach ist die Implementierung von einem grundlegenden Sensorsystem in die Instrumentenhalterung bzw. Instrumentenhalterungen, die dazu vorgesehen und ausgebildet ist/ sind, Daten erfassen und zwischenspeichern zu können, von Vorteil. Unter einem vollständigen Sterilisationskreislauf sind unter anderem die folgenden Phasen/ Prozessschritte zu verstehen:
- Ultraschallreinigung oder manuelle Vorreinigung.
- Reinigung im RDG (Reinigungs- und Desinfektionsgerät);
- Wartung (zum Beispiel eine Öl-Durchsprühung); und
- Sterilisation.

Es ist vorgesehen, wenn in der zentralen Elektronikeinheit zumindest ein Verteilermodul/- Multiplexer vorgesehen und ausgebildet ist, das/ der dazu konfiguriert ist, um jeweils zumindest zwei Halterungssysteme über das jeweilige Antennensystem anzusteuern, wobei jedes Verteilermodul dazu konfiguriert ist, über analoge und digitale Eingänge zu kommunizieren. Unter einem Verteilermodul bzw. Multiplexer ist eine Selektionsschaltung in der analogen und digitalen Elektronik zu verstehen, mit der aus einer Anzahl von Eingangssignalen ausgewählt und an den Ausgang durchgeschaltet werden kann. Dadurch wird ermöglicht, dass mehrere Antennensysteme, wobei jedes Antennensystem eine oder mehrere Antennen aufweisen kann, mit der zentralen Elektronikeinheit verbunden sind und mit dieser kommunizieren können. Das Verteilermodul verfügt über Antennenschnittstellen und stellt somit die Kontaktierung/ Verbindung zwischen der zentralen Elektronikeinheit und den Antennen- und Datenverbindungen bzw. der Leiterbahnen mit den daran angeschlossenen Antennen bzw. Antennensystemen des Halterungssystems bzw. der Halterungssysteme dar. Durch die Verwendung eines solchen Verteilermoduls kann eine große Anzahl an Antennen angesteuert werden, ohne dass eine Vielzahl an Leitungen im Siebkorb untergebracht werden muss.

Ferner ist es bevorzugt, wenn mehrere Verteilermodule derart vorgesehen und ausgebildet sind, um in einer Reihenschaltung mit der zentralen Elektronikeinheit verbunden/verbindbar zu sein, um die jeweiligen Verteilermodule hintereinander anzusteuern und um so das zumindest eine jeweilige Antennensystem durchzuschalten, um das auf dem zumindest einen jeweiligen Antennensystem liegende Sterilgut auszulesen.

Bevorzugterweise ist das zumindest eine jeweilige Antennensystem der vorbestimmten Anzahl an Halterungssystemen als NFC-Antenne vorgesehen und ausgebildet ist. Die Verwendung einer NFC-Antenne hat den Vorteil, dass eine unmittelbare Nähe des Sterilguts zur Antenne notwendig ist, und somit die Fehlerquote bzw. ein Fehlinventarisieren geringer/unwahrscheinlicher ist. Alternativ ist auch die Verwendung einer RFID-Antenne möglich.

Bei einer vorteilhaften Ausgestaltung der Erfindung ist vorgesehen, dass die vorbestimmte Anzahl an Halterungssystemen jeweils mit einem Sensorsystem zum Erfassen und Zwischenspeichern von Daten vorgesehen und ausgebildet ist, wobei das Sensorsystem zumindest einen Sensor, eine Datenverarbeitungseinheit, eine Kommunikationseinheit, einen temporären Speicher und eine wiederaufladbare Energiespeichereinheit hat. Unter einem temporären Speicher ist ein Speicher zu verstehen, welcher Daten/ Informationen oder Ähnliches zeitlich begrenzt speichert, welche von dem gleichen oder einem anderen Programm weiterverarbeitet werden sollen. Hierbei gilt, dass von dem Sensorsystem erfasste Prozessschritte und Sensorwerte zwischengespeichert werden und an die zentrale Elektronikeinheit übertragen werden, sobald das jeweilige Halterungssystem mit der zentralen Elektronikeinheit verbunden ist. Parallel zu dem Datenaustausch ist es bevorzugt vorgesehen, dass die Energiespeichereinheit aufgeladen wird.

Es ist bevorzugt, wenn die zentrale Elektronikeinheit zum Erfassen und Zwischenspeichern von Daten vorgesehen und ausgebildet ist und in Kommunikationsverbindung mit dem Sensorsystem der vorbestimmten Anzahl an Halterungssystemen und/ oder mit dem in der vorbestimmten Anzahl an Halterungssystemen gehaltenen Sterilguts zu stehen.

Es ist von Vorteil, wenn die Datenverarbeitungseinheit dazu vorgesehen und angepasst ist, um Daten zu erfassen und in die vorbestimmte Anzahl an Halterungssystemen und/oder in das von der vorbestimmten Anzahl an Halterungssystemen gehaltene Sterilgut zu schreiben.

In anderen Worten kann die Datenverarbeitungseinheit ermittelte Reinigungsinformationen/Daten, welche beispielsweise über die Umgebung erfasst werden, in einem Datenspeicher der Halterungsvorrichtung bzw. in einem RFID-Tag des von dem Halterungssystem gehaltenen Sterilguts geschrieben und zwischengespeichert werden. Daher besteht eine Kommunikationsverbindung mit der Datenverarbeitungseinheit, welche einen internen Datenspeicher aufweist, so dass die Datenverarbeitungseinheit nach Verarbeitung der Ausgabesignale die Zyklusinformationen des Sterilgutkreislaufs in den internen Datenspeicher der Halterungsvorrichtung bzw. des Sterilguts schreiben kann. Das hat den Vorteil, dass die Elektronikeinheit als Puffer/Zwischenspeicher vorgesehen ist, um bspw. Daten zeitweise zu speichern, wenn die für diese Daten entsprechende Halterungsvorrichtung bzw. das entsprechende Sterilgut nicht verfügbar ist. Das Erfassungssystem ist dann in der Lage, die Daten zu einem späteren Zeitpunkt, wenn die entsprechende Halterungsvorrichtung bzw. das entsprechende Sterilgut wieder verfügbar ist, mit den Daten zu beschreiben. Auf diese Weise ist es möglich, sich von einer ständigen Netzwerkverbindung bzw. Cloudlösung zu entfernen.

Zusammenfassend ermöglicht die Verwendung von jeweils einem Sensorsystem in den Halterungssystemen eine Erfassung von Prozessschritten, ohne dass die gesamte Elektronikeinheit angeschlossen sein muss. Dies hat den Vorteil, dass auch eine individuelle Reinigung möglich ist.

Es ist vorteilhafterweise auch vorgesehen, wenn das modulare Erfassungssystem dazu vorgesehen und ausgebildet ist, um mit zumindest einem weiteren modularen Erfassungssystem zu kommunizieren und Daten auszutauschen. Auf diese Weise ist es möglich, dass zumindest zwei unabhängige modulare Erfassungssysteme mit jeweils einer zentralen Elektronikeinheit über diese zentralen Elektronikeinheiten miteinander kommunizieren und untereinander Daten austauschen. Dies erfolgt vorzugsweise mittels einem proprietären Protokoll, wahlweise über jegliche Funktechnologie. Somit kann ein ganzes Mesh/ Netz von modularen Erfassungssystemen bzw. von den jeweiligen Elektronikeinheiten der modularen Erfassungssysteme aufgebaut werden, ein sogenanntes eigenständiges Firmen-Mesh/ Netz, welches volle Informationstransparenz bietet. Unter einem proprietären Protokoll ist ein Protokoll zu verstehen, das das Recht und die Möglichkeit der Wieder- und Weiterverwendung sowie Änderung und Anpassung durch Nutzer und Dritte stark einschränkt.

Ferner ist es bevorzugt, wenn die zentrale Elektronikeinheit dazu vorgesehen und ausgebildet ist, um über das zumindest eine Verteilermodul/ den einen Multiplexer das zumindest eine jeweilige Antennensystem der festgelegten Anzahl an Halterungssystemen auszulesen und die erhaltenen Daten und/ oder Informationen an eine externe Einheit, vorzugsweise ein Smartphone und/oder eine App und/ oder ein Steuergerät, zu übertragen, welche dazu vorgesehen und ausgebildet ist, die erhaltenen Daten und/ oder Informationen, vorzugsweise Artikelnummer, Seriennummer, Antennenplatz, Lageplatz und/ oder Aufbereitungszyklen, visuell auszugeben.

Ferner ist es von Vorteil, wenn die verbaute Elektronik gegen die Temperaturen und gegen Reinigungsmittel, insbesondere Feuchtigkeit und alkalische Reiniger, geschützt ist. Hierbei ist ein einfaches Gehäuse mit einer Isolierung/ Isolation zum Überbrücken der Hochtemperaturphase > 130°C für etwa 10 Minuten im Sterilisator fundamental. Ein solcher Schutz kann über dicke Isolationsschichten in dem Gehäuse als auch Beschichtungen der Elektronik selbst erreicht werden. Ebenso ist ein Verguss der Elektronik denkbar.

Ferner betrifft die vorliegende Erfindung ein Halterungssystem zum Halten von Sterilgut, insbesondere von chirurgischen Instrumenten und/ oder Motorensystemen und/ oder Medizinprodukten. Hierbei ist es vorgesehen, wenn das Halterungssystem ein Sensorsystem zum eigenständigen Erfassen von Prozessschritten aufweist, wobei das Halterungssystem dazu vorgesehen und ausgebildet ist, an ein modulares Erfassungssystem gemäß einem der vorstehenden Aspekte angeschlossen zu sein.

Ferner können in anderen Worten ausgedrückt, auf diesen Einsatz für den Siebkorb unterschiedliche Halterungssysteme für Instrumente und Motorensystem aufgesetzt werden, die mit den vorhandenen Schnittstellen verbunden sind. Diese Halterungssysteme haben wiederum NFC-Antennen implementiert, mit denen NFC-Transponder auf den Medizinprodukten/ dem Sterilgut ausgelesen werden können. Weiterhin befindet sich jeweils eine Sensoreinheit/ ein Sensorsystem in den jeweiligen Halterungssystemen, die über einen Sensor, eine Datenverarbeitungseinheit, einen temporären Speicher und einen wiederaufladbaren Energiespeicher verfügt. Sofern mehr als eine Antenne verwendet wird, befindet sich in der zentralen Elektronikeinheit zudem mindestens ein Multiplexer.

Ferner betrifft die vorliegende Erfindung ein Siebkorbsystem zum Überwachen und Rückverfolgen von Sterilgut, insbesondere von chirurgischen Instrumenten und/ oder Motorensystemen und/ oder Medizinprodukten, in einem vollständigen Sterilgutkreislauf. Demzufolge ist es bevorzugt, wenn ein erster und zumindest ein weiterer Siebkorb gestapelt/ stapelbar vorgesehen und ausgebildet sind, in welche jeweils ein modulares Erfassungssystem gemäß einem der vorstehenden Aspekte eingelegt/ einlegbar ist, wobei nur das modulare Erfassungssystem des ersten Siebkorbs die zentrale Elektronikeinheit aufweist, welche mit dem modularen Erfassungssystem des zumindest einen weiteren Siebkorbs ohne dem Modul der zentralen Elektronikeinheit, vorzugsweise über weitere Verteilermodule/ Multiplexer, verbunden/ verbindbar ist.

In anderen Worten können mehrere Siebe/ Siebkörbe, die im Verbund immer zusammenbleibend vorgesehen sind, gestapelt werden. Hier ist allerdings vorgesehen, dass nur in einem ersten Siebkorb, welches auch als Hauptsieb bezeichnet wird, die zentrale Elektronikeinheit eingebracht wird. Die Konnektierung auf die weiteren Siebe/Siebkörbe und deren Antennen bzw. Antennensysteme, kann dann mittels Kontakten/Schnittstellen und Verteilermodulen hergestellt werden.

Ferner betrifft die vorliegende Erfindung ein Verfahren zur Überwachung eines Sterilgutkreislaufs unter Verwendung zumindest eines modularen Erfassungssystems gemäß einem der vorstehenden Aspekte, mit den folgenden Schritten:
- Erfassen und Zwischenspeichern von Prozessschritten und/ oder Sensorwerten von zumindest einem Halterungssystem;
- Anschließen/ Verbinden des zumindest einen Halterungssystems mit der zentralen Elektronikeinheit des modularen Erfassungssystems über zumindest eine Daten- und Antennenschnittstelle;
- Übertragen von Daten, insbesondere der Prozessschritte und der Sensorwerte, des zumindest einen Halterungssystems an die Datenverarbeitungseinheit des modularen Erfassungssystems;
- Aufladen der Energiespeichereinheit;
- Auswerten und Evaluieren der Daten in der Datenverarbeitungseinheit des modularen Erfassungssystems;
- Auslesen einer Instrumenten-ID zum Abgleichen mit einer hinterlegten Liste für das zumindest eine angeschlossene/ verbundene Halterungssystem; und
- Informationsausgabe bei einem negativen Abgleichergebnis.

In anderen Worten werden sobald das zumindest eine Halterungssystem mit der zentralen Elektronikeinheit verbunden ist, die gesammelten Daten übertragen und der Energiespeicher aufgeladen. Anschließend werden die Daten mit einer effizienten Datenverarbeitungseinheit weiter ausgewertet und evaluiert. Hierbei ist eine weitere Aufgabe die Überprüfung der korrekten Instrumentenplatzierungen. Dies erfolgt durch das Auslesen der einzelnen in den Halterungssystemen bzw. dem Halterungssystem angebrachten Instrumente.

Zusammenfassend betrifft die Erfindung einen Einsatz für einen Siebkorb, der eine zentrale Elektronikeinheit besitzt, die über Sensoren, Energiespeicher, Kommunikationsmodule und eine NFC-Schreib-/ Ausleseeinheit verfügt. Weiterhin besitzt der Einsatz Daten- und Antennenverbindungen, die zu einer definierten Anzahl an Steckplätzen führen, an die intelligente/ smarte Halterungssysteme wahlweise angeschlossen sind, die jeweils über NFC-Antennen verfügen. Zudem verfügen die einzelnen Halterungssysteme über eine einfache Sensoreinheit, die Daten zwischenspeichern und weitergeben kann. Somit kann das Sieb/ der Siebkorb und dessen Sterilgut/Produkt in Echtzeit inventarisiert werden und ist durch eine Echtzeiterkennung interaktiv. So kann dem Anwender unmittelbar das entsprechende Sterilgut/ Produkt mit Seriennummer und Lebensdauer zurückgemeldet werden. Eine Entnahme des Sterilguts/ Produkts wird somit erkannt und entsprechend geloggt/ aufgezeichnet.

Das System überwacht mit den vorhandenen Sensoren die Umgebung und erkennt automatisch, ob eine Ultraschall-Reinigung, eine Reinigung in dem RDG, eine Wartung, beispielsweise eine Öl-Durchsprühung, oder eine Sterilisation durchgeführt wird. Hierbei ist die Erweiterung der Bandbreite an weiteren Sensoriken möglich. In dem Fall, dass ein Halterungssystem von dem Siebkorb abgekoppelt ist, speichert die in dem Halterungssystem verbaute Sensoreinheit die Daten und gibt diese nach einer erneuten Verbindung mit der zentralen Elektronikeinheit in dem Siebkorb weiter, damit eine Auswertung stattfinden kann. Weiterhin ist es möglich, dass jederzeit überprüft wird, ob ein Instrument im richtigen Halterungssystem untergebracht wurde, und dies in Echtzeit. Hierfür werden die verbauten Transponder in dem Sterilgut ausgelesen und mit einer gespeicherten Liste verglichen. Die Identifikation des Halterungssystems erfolgt durch die verbaute Datenverarbeitungseinheit. Die Auswertung kann entweder durch die zentrale Datenverarbeitungseinheit erfolgen oder mittels Bluetooth, WiFi oder jedem anderen Funkstandard, und an eine externe Recheneinheit gesendet und ausgewertet werden.

### Kurzbeschreibung der Figuren

Fig. 1 ist eine Darstellung zur Veranschaulichung eines modularen Erfassungssystems gemäß der vorliegenden Offenbarung;
Fig. 2 ist eine seitliche Explosionsansicht des modularen Erfassungssystems gemäß der vorliegenden Offenbarung;
Fig. 3 ist eine weitere Explosionsansicht des modularen Erfassungssystems gemäß der vorliegenden Offenbarung;
Fig. 4 ist eine Darstellung zur Veranschaulichung des Siebkorbs mit dem auf einer Basisplatte angeordneten modularen Erfassungsmodul gemäß der vorliegenden Offenbarung;
Fig. 5 ist eine Darstellung zur Veranschaulichung des auf einer Basisplatte angeordneten modularen Erfassungsmoduls gemäß der vorliegenden Offenbarung;
Fig. 6 ist eine Darstellung zur Veranschaulichung des direkt in den Siebkorb eingelegten modularen Erfassungsmoduls gemäß der vorliegenden Offenbarung;
Fig. 7 ist eine Darstellung zur Veranschaulichung des modularen Erfassungsmoduls gemäß der vorliegenden Offenbarung;
Fig. 8 ist eine Darstellung zur Veranschaulichung des modularen Erfassungsmoduls bestehend aus einer zentralen Elektronikeinheit und den Leiterbahnen gemäß der vorliegenden Offenbarung;
Fig. 9 ist eine Darstellung zur Veranschaulichung des auf der Basisplatte angeordneten modularen Erfassungsmoduls bestehend aus einer zentralen Elektronikeinheit und den Leiterbahnen gemäß der vorliegenden Offenbarung;
Fig. 10 ist eine Darstellung zur Veranschaulichung der Unterseite des auf der Basisplatte angeordneten modularen Erfassungsmoduls bestehend aus einer zentralen Elektronikeinheit und den Leiterbahnen gemäß der vorliegenden Offenbarung;
Fig. 11 ist eine Darstellung zur Veranschaulichung eines Halterungssystems mit einer Antenne gemäß der vorliegenden Offenbarung;
Fig. 12 ist eine Darstellung zur Veranschaulichung der Unterseite des Halterungssystems mit einer Antenne und zwei Antennenanschlüssen gemäß der vorliegenden Offenbarung;
Fig. 13 ist eine Darstellung zur Veranschaulichung eines Halterungssystems mit einem Antennensystem gemäß der vorliegenden Offenbarung;
Fig. 14 ist eine Darstellung zur Veranschaulichung der Unterseite des Halterungssystems mit einem Antennensystem und einem Antennenanschluss gemäß der vorliegenden Offenbarung;
Fig. 15 ist eine Darstellung zur Veranschaulichung eines Systemaufbaus des modularen Erfassungsmoduls gemäß der vorliegenden Offenbarung;
Fig. 16 ist eine Darstellung zur Veranschaulichung eines einfachen Systemaufbaus des modularen Erfassungsmoduls mit einem Verteilermodul und zumindest einer externen Einheit gemäß der vorliegenden Offenbarung;
Fig. 17 ist eine Darstellung zur Veranschaulichung des Systemaufbaus des modularen Erfassungsmoduls mit mehreren Verteilmodulen gemäß der vorliegenden Offenbarung;
Fig. 18 ist eine Darstellung zur Veranschaulichung der Kommunikation zwischen zwei einfachen Systemaufbauten des modularen Erfassungsmoduls mit jeweils einem Verteilermodul gemäß der vorliegenden Offenbarung;
Fig. 19 ist eine Darstellung zur Veranschaulichung des Mesh-Netzwerks im Vergleich zu einem herkömmlichen Netzwerk gemäß der vorliegenden Offenbarung;
Fig. 20 ist eine Darstellung eines beispielhaften Temperatur- und Druckverlaufs in einem Sterilisator gemäß der vorliegenden Offenbarung.

### Beschreibung der Ausführungsbeispiele

Nachstehend werden bevorzugte Ausführungsbeispiele der vorliegenden Offenbarung auf der Basis der zugehörigen Figuren beschrieben.

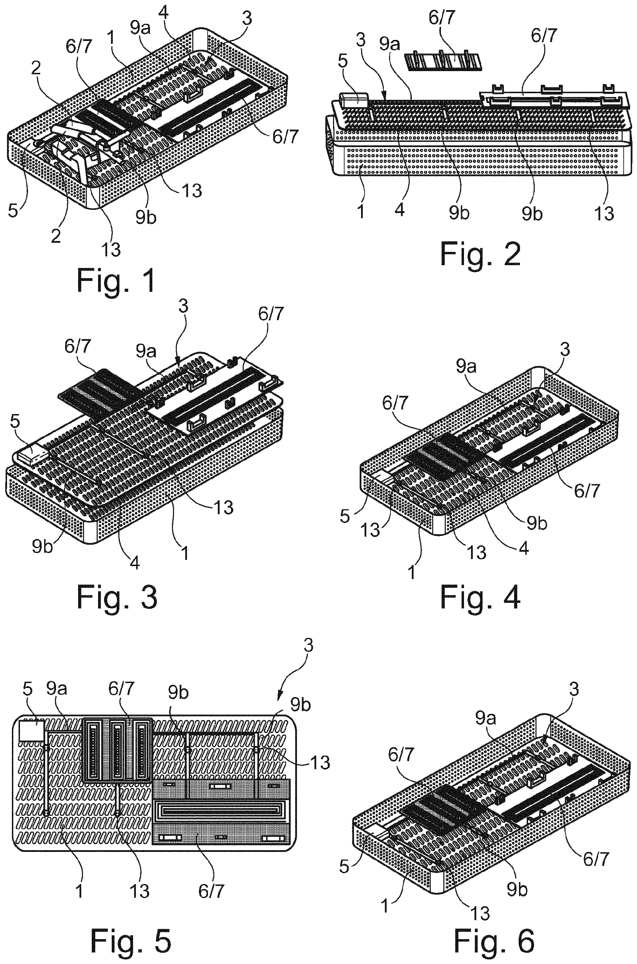

Fig. 1 ist eine Darstellung zur Veranschaulichung eines modularen Erfassungssystems gemäß einem ersten Ausführungsbeispiel der vorliegenden Offenbarung. Fig. 1 zeigt das modulare Erfassungssystem zum Einlegen in einen Siebkorb 1 zum Erfassen, Überwachen und Rückverfolgen von Sterilgut 2. Das modulare Erfassungssystem ist als ein Siebkorbeinsatz 3 vorgesehen und ausgebildet, der gemäß einem zweiten Ausführungsbeispiel (siehe Fign. 5 und 6) direkt oder gemäß einem ersten Ausführungsbeispiel (siehe Fign. 1 bis 4) auf einer Basisplatte 4 angeordnet, in den Siebkorb 1 einlegbar ist. In Fig. 1 ist eine zentrale Elektronikeinheit 5, eine definierte Anzahl an Steckplätzen 6, eine vorbestimmte Anzahl an Halterungssystemen 7 (hier zwei Halterungssysteme) und eine festgelegte Anzahl an Leiterbahnen 9 gezeigt.

In Fig. 1 ist gezeigt, dass die Basisplatte 3, welche vorzugweise die gleiche Größe und Form wie die Grundfläche des Siebkorbs 1 aufweist, in den Siebkorb 1 eingelegt/ einlegbar ist. Auf der Basisplatte 3 ist die zentrale Elektronikeinheit 5 angeordnet, welche mit den Leiterbahnen 9 verbunden ist, welche wiederum über zumindest einen Antennen- und Datenanschluss 13 mit zumindest einem platten-/ platinenförmigen Halterungssystem 7 verbindbar/ verbunden ist. Die Fign. 2 und 3 zeigen entsprechende Explosionsansichten von Fig. 1, und Fig. 4 entspricht im Wesentlichen der Fig. 1 ohne dem Sterilgut 2. Somit werden die Fign. 2 bis 4 nicht weiter beschrieben.

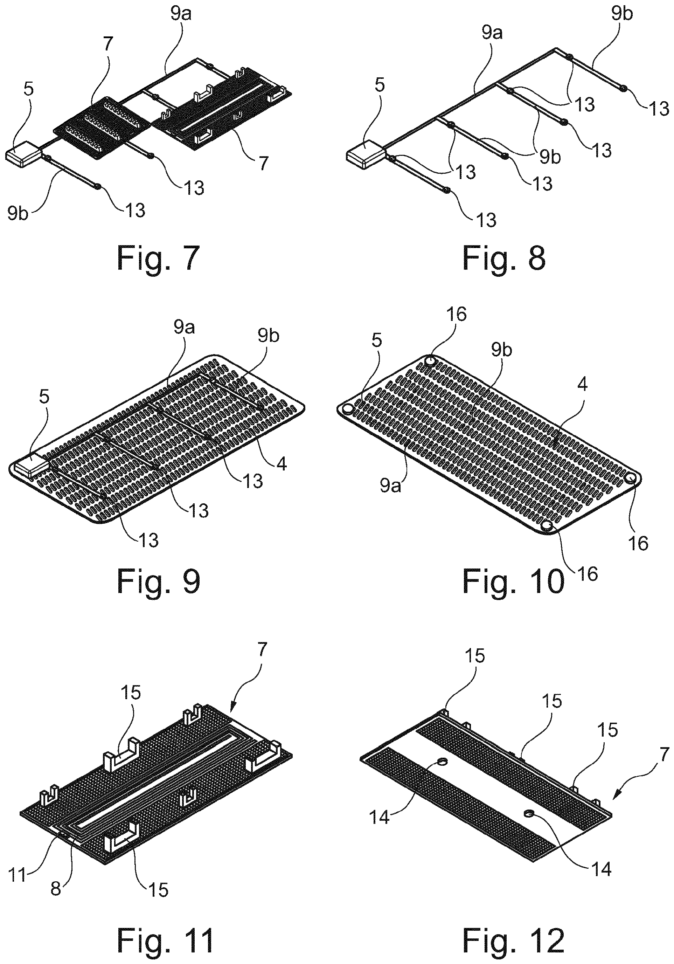

Fig. 1 zeigt in Verbindung mit den Fign. 8 und 9 die Leiterbahnen 9 in Verbindung mit der zentralen Elektronikeinheit 5, welche vorzugsweise den in den Figuren gezeigten Leiterbahnenaufbau haben sollen. Dabei ist es vorgesehen, dass die Leiterbahnen 9b von einer mit der zentralen Elektronikeinheit 5 direkt verbundenen Leiterbahn 9a im rechten Winkel abzweigen. Jede Leiterbahn 9b endet jeweils in einem Antennen- und Datenanschluss 13, wobei ferner bevorzugt ist, dass auf einer Leiterbahn 9b noch zumindest ein weiterer Antennen- und Datenanschluss 13 vorgesehen ist. Ferner ist es bevorzugt, wenn genau vier von der Leiterbahn 9a abzweigende Leiterbahnen 9b vorgesehen sind, wobei auch weniger oder mehr Leiterbahnen 9b vorgesehen sein können.

Fig. 1 zeigt in Verbindung mit den Fign. 11 bis 14 die Halterungssysteme 7, welche an den Steckplätzen 6 angebracht sind, wobei sich die Steckplätze je nach Halterungssystem 7 über die erforderliche Anzahl an Antennen- und Datenanschlüssen 13 definieren. Gemäß der vorliegenden Ausführungsbeispiele sind zwei unterschiedlich ausgebildete Halterungssysteme 7 in dem Siebkorb 1 vorgesehen, wobei jedes Halterungssystem 7 mit einer Sensoreinheit 11 ausgebildet ist, in welcher Daten und Sensorwerte erfasst und zwischengespeichert werden und welche nach Verbinden mit der zentralen Elektronikeinheit 5 an diese übertragen werden.

Gemäß den Fign. 11 und 12 ist ein Halterungssystem 7 vorgesehen, welches mit einem Antennensystem 8 bestehend aus einer Antenne ausgebildet ist.

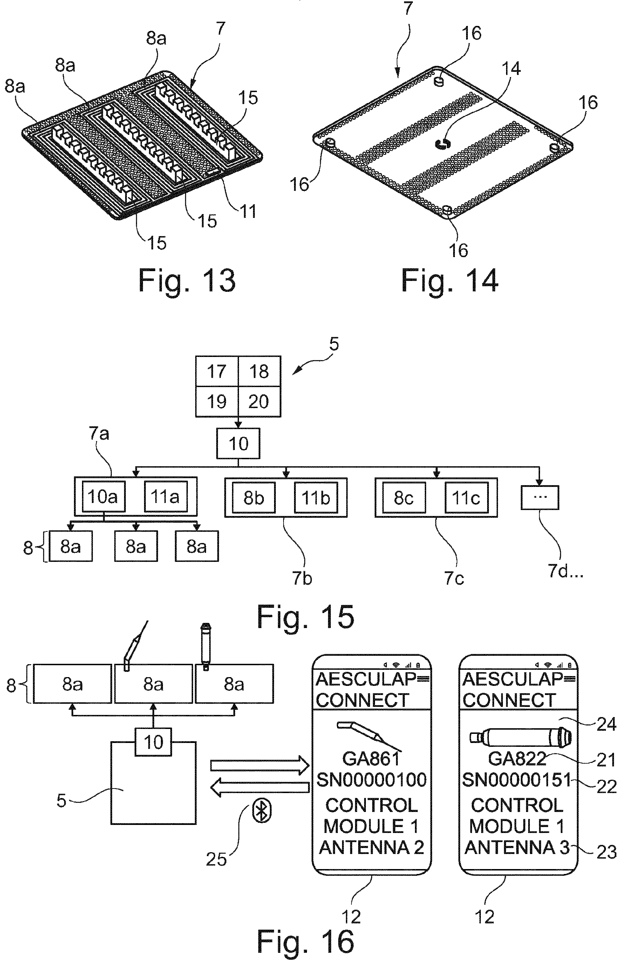

Hierbei zeigt Fig. 11 eine Oberseite eines solchen platinenartigen Halterungssystems 7, auf welcher zumindest eine Sterilgutauflagerung (Sockel/ Klemme) 15 ausgebildet angeordnet ist, in welche das Sterilgut 2 gemäß Fig. 1 eingelegt werden kann. Fig. 12 zeigt eine Unterseite eines solchen Halterungssystems 7, in welcher die zu den Antennen- und Datenanschlüssen 13 der Leiterbahnen 9 kompatiblen Antennenanschlüsse 14, einem sogenannten doppelten Anschluss, abgebildet sind. Die Halterungssysteme 7 können mittels diesem zu einem Antennen- und Datenanschluss 13 kompatiblen Antennenanschluss 14 (siehe Fig. 12 und 14) an den Leiteraufbau angebracht bzw. auf dem Leiteraufbau befestigt werden. In Fig. 14 ist ferner gezeigt, dass an der Unterseite des Halterungssystems 7 zusätzlich zu den Antennenanschlüssen 14 an jeder Ecke ein Auflagemittel 16 vorgesehen ist, welches zum Auflegen auf und bevorzugterweise zum Befestigen an der Basisplatte 3 bzw. dem Siebkorb 1 vorgesehen ist.

Gemäß der Fig. 13 ist ein Halterungssystem 7 mit einem Antennensystem 8, welches mit drei Antennen 8a ausgebildet ist, vorgesehen. Das Halterungssystem 7 weist ebenfalls eine Sensoreinheit 11 auf, welche mit einem Verteilermodul 10 (nicht gezeigt) ausgebildet ist, um jede der drei Antennen 8a anzusprechen.

Die Fign. 5 bis 7 sind eine Darstellung zur Veranschaulichung des modularen Erfassungsmoduls gemäß dem zweiten Ausführungsbeispiel ohne Basisplatte 3 und entspricht ansonsten im Wesentlichen dem Aufbau des vorstehend beschriebenen ersten Ausführungsbeispiels. Auf eine wiederholte Beschreibung wird daher an dieser Stelle verzichtet.

Die Fign. 9 und 10 sind eine Darstellung zur Veranschaulichung der Oberseite und Unterseite des auf der Basisplatte 3 angeordneten modularen Erfassungsmoduls bestehend aus einer zentralen Elektronikeinheit 5 und den Leiterbahnen 9 gemäß der vorliegenden Offenbarung. In Fig. 10 ist ferner gezeigt, dass an der Unterseite der Basisplatte 3 weitere Auflagemittel (Abstandshalter/ Rastfüße/ etc.) 16 zum Einlegen und bevorzugterweise zum Befestigen in den/dem Siebkorb 1 vorgesehen sind.

Fig. 15 ist eine Darstellung zur Veranschaulichung eines Systemaufbaus des modularen Erfassungsmoduls gemäß der vorliegenden Offenbarung. In Fig. 5 ist die zentrale Elektronikeinheit 5 gezeigt, in welcher eine Energiespeichereinheit 17, eine Datenverarbeitungseinheit 18, ein Kommunikationsmodul 19 und eine Ausleseeinheit 20 vorgesehen sind. Die zentrale Elektronikeinheit 5 ist über ein Verteilermodul 10 mit mehreren Halterungssystemen 7 verbunden. Hierbei hat das erste Halterungssystem 7a ein Sensorsystem 11a und ein weiteres Verteilermodul 10a, welches mit einem Antennensystem 8 verbunden ist, wobei das Antennensystem 8 aus mehreren Antennen 8a besteht, die jeweils von dem Verteilermodul 10a angesprochen werden. Ferner ist ein zweites Halterungssystem 7b und ein drittes Halterungssystem 7c vorgesehen, welche jeweils ein Antennensystem mit mehreren Antennen 8b bzw. 8c und eine Sensoreinheit 11b bzw. 11c aufweisen und jeweils von dem ersten Verteilermodul 10 angesprochen werden. Die Anzahl an Halterungssystemen 7 sowie die Antennensysteme bzw. Antennen 8 ist modular beliebig erweiterbar.

Fig. 16 ist eine Darstellung zur Veranschaulichung eines einfachen Systemaufbaus des modularen Erfassungsmoduls mit einem Verteilermodul 10 und zumindest einer externen Einheit 12 gemäß der vorliegenden Offenbarung. Hierbei steuert die zentrale Elektronikeinheit 5 über ein Verteilmodul 10 die jeweiligen Antennen 8a des Antennensystems 8 an und liest diese aus. Diese Information wird an eine externe Einheit 12 weiterkommuniziert. In dem dargestellten Fall ist die externe Einheit 12 eine App auf einem Smartphone, welche die Informationen über Artikelnummer 21, Seriennummer 22, Antennenplatz 23 respektive Lagerplatz visuell auf einem Display 24 ausgibt. Weitere Daten wie Aufbereitungszyklen etc. sind hinzufügend anzeigbar. Ein bevorzugtes Übertragungsmittel, vorzugsweise bidirektional ausgebildet, ist in diesem Fall Bluetooth bzw. jeglicher andere Funkstandard.

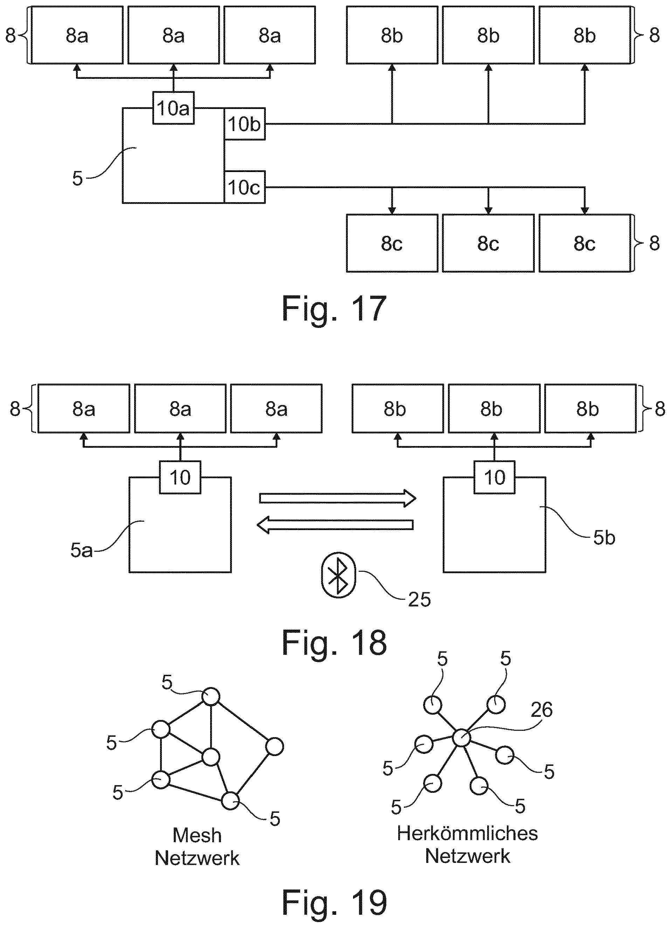

Fig. 17 ist eine Darstellung zur Veranschaulichung des Systemaufbaus des modularen Erfassungsmoduls mit mehreren Verteilmodulen 10 gemäß der vorliegenden Offenbarung. Fig. 17 zeigt die zentrale Elektronikeinheit 5, welche die drei Verteilmodule 10a, 10b und 10c hintereinander ansteuert. Jedes Verteilermodul 10a, 10b und 10c schaltet wiederum ein Antennensystem 8 mit den jeweils drei Antennen 8a, drei Antennen 8b und drei Antennen 8c durch. So wird ausgegeben, welches Sterilgut 2 (Medizinprodukte bzw. Instrumente und/ oder Motorensysteme) auf den jeweils drei Antennen 8a, den jeweils drei Antennen 8b und den jeweils drei Antennen 8c liegt. Auf diese Art und Weise können sehr komplexe Siebe/ Siebkörbe 1 inventarisiert werden. Hierbei ist besonders bevorzugt, von der unmittelbaren Nähe der Tags zur Antenne zu profitieren und so ein Fehlinventarisieren zu unterbinden.

Fig. 18 ist eine Darstellung zur Veranschaulichung der Kommunikation zwischen zwei einfachen Systemaufbauten des modularen Erfassungsmoduls mit jeweils einem Verteilermodul 10 gemäß der vorliegenden Offenbarung. Fig. 18 zeigt zwei zentrale Elektronikeinheit 5a und 5b, welche über das Übertragungsmittel 25, vorzugsweise Bluetooth, untereinander/ miteinander kommunizieren und Daten austauschen können. Die zentralen Elektronikeinheiten 5a und 5b weisen jeweils ein Verteilermodul 10 auf, welches wiederum jeweils ein Antennensystem 8 mit jeweils drei Antennen 8a und 8b anspricht/ ansteuert.

Fig. 19 ist eine Darstellung zur Veranschaulichung des Mesh-Netzwerks gemäß der vorliegenden Offenbarung im Vergleich zu einem herkömmlichen Netzwerk. Hierbei ist auf der rechten Seite der Fig. 19 ein herkömmliches Netzwerk gezeigt, in welchem alle Erfassungssysteme, hier vereinfacht als Elektronikeinheiten 5 bezeichnet, an einem zentralen Vermittler 26 zusammenlaufen und eine direkte Kommunikation zwischen den Elektronikeinheiten 5 nicht vorgesehen ist. Auf der linken Seite der Fig. 19 ist Mesh-Netzwerk gemäß der vorliegenden Offenbarung darstellt, in welchem die direkte Kommunikation zwischen den Elektronikeinheiten 5 möglich ist und so kein Vermittler mehr erforderlich ist.

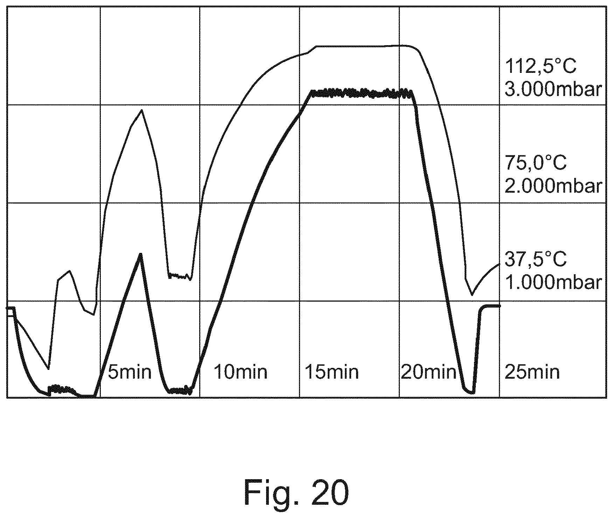

Fig. 20 ist eine Darstellung eines beispielhaften Temperatur- und Druckverlaufs in einem Sterilisator gemäß der vorliegenden Offenbarung. Hierbei stellt die obere Kurve den Temperaturverlauf (Y-Achse) und die untere Kurve den Druckverlauf über der Zeit (x-Achse) in einem Sterilisator dar.

### Bezugszeichen

- 1: Siebkorb
- 2: Sterilgut
- 3: Siebkorbeinsatz
- 4: Basisplatte
- 5: Elektronikeinheit
- 6: Steckplatz
- 7: Halterungssystem
- 8: Antennensystem
- 9: Leiterbahn
- 10: Verteilermodul
- 11: Sensorsystem
- 12: externe Einheit
- 13: Antennen- und Datenanschluss
- 14: Antennenanschluss
- 15: Sterilgutauflagerung
- 16: Auflagemittel
- 17: Energiespeichereinheit
- 18: Datenverarbeitungseinheit
- 19: Kommunikationsmodul
- 20: Ausleseeinheit
- 21: Artikelnummer
- 22: Seriennummer
- 23: Antennenplatz
- 24: Display
- 25: Übertragungsmittel
- 26: Vermittler

## Patentansprüche

1. Modulares Erfassungssystem zum Einlegen in einen Siebkorb (1) zum Erfassen, Überwachen und Rückverfolgen von Sterilgut (2), insbesondere von chirurgischen Instrumenten und/oder Motorensystemen und/oder Medizinprodukten in einem Sterilisationskreislauf, wobei das modulare Erfassungssystem als ein Siebkorbeinsatz (3) vorgesehen und ausgebildet ist, der direkt oder auf einer Basisplatte (4) angeordnet in den Siebkorb (1) einlegbar ist, mit individuell und wahlweise elektrisch koppelbaren folgenden Modulen:
einer zentralen Elektronikeinheit (5), die vorzugsweise mit zumindest einem Sensor, einer Energiespeichereinheit (17), einer Datenverarbeitungseinheit (18), einer Kommunikationseinheit (19) und einer Schreibe- und Ausleseeinheit (20), vorzugsweise mittels NFC, ausgebildet ist,
einer definierten oder freiwählbaren Anzahl an Steckplätzen (6) zum Anschließen und/ oder Verbinden einer vorbestimmten Anzahl an Halterungssystemen (7) zum Halten von Sterilgut (2), die mit jeweils zumindest einem Antennensystem (8) zum Auslesen eines Transponders, vorzugsweise eines NFC-Transponders, des Sterilguts (2) vorgesehen und ausgebildet ist, und
einer vorzugsweise festgelegten Anzahl an Leiterbahnen (9), insbesondere Daten- und Antennenleitungen und/oder -verbindungen, welche derart vorgesehen und ausgebildet sind, um die Steckplätze (6) und/oder Halterungssysteme (7) miteinander und/ oder mit der zentralen Elektronikeinheit (5) elektrisch zu verbinden.

2. Modulares Erfassungssystem gemäß Anspruch 1, **dadurch gekennzeichnet, dass** in der zentralen Elektronikeinheit (5) zumindest ein Verteilermodul (10) vorgesehen und ausgebildet ist, das dazu konfiguriert ist, um jeweils zumindest zwei Halterungssysteme (7) über das jeweilige Antennensystem (8) anzusteuern.

3. Modulares Erfassungssystem gemäß einem der vorstehenden Ansprüche,
**dadurch gekennzeichnet, dass** mehrere Verteilermodule (10) derart vorgesehen und ausgebildet sind, um in einer Reihenschaltung mit der zentralen Elektronikeinheit (5) verbunden/verbindbar zu sein, um die jeweiligen Verteilermodule (10) hintereinander anzusteuern und so das zumindest eine jeweilige Antennensystem (8) durchzuschalten, um das auf dem zumindest einen jeweiligen Antennensystem (8) liegende Sterilgut (2) auszulesen.

4. Modulares Erfassungssystem gemäß einem der vorstehenden Ansprüche,
**dadurch gekennzeichnet, dass** das zumindest eine jeweilige Antennensystem (8) der Anzahl an Halterungssystemen (7) als NFC-Antenne vorgesehen und ausgebildet ist.

5. Modulares Erfassungssystem gemäß einem der vorstehenden Ansprüche,
**dadurch gekennzeichnet, dass** die Anzahl an Halterungssystemen (7) jeweils mit einem Sensorsystem (11) zum Erfassen und Zwischenspeichern von Daten vorgesehen und ausgebildet ist, wobei das Sensorsystem (11) zumindest einen Sensor, eine Datenverarbeitungseinheit, einen temporären Speicher und eine wiederaufladbare Energiespeichereinheit hat.

6. Modulares Erfassungssystem gemäß einem der vorstehenden Ansprüche,
**dadurch gekennzeichnet, dass** die zentrale Elektronikeinheit (5) zum Erfassen und Zwischenspeichern von Daten vorgesehen und ausgebildet ist und in Kommunikationsverbindung mit dem Sensorsystem (11) der vorbestimmten Anzahl an Halterungssystemen (7) und/ oder mit dem in der vorbestimmten Anzahl an Halterungssystemen (7) gehaltenen Sterilguts (2) zu stehen.

7. Modulare Erfassungssystem gemäß einem der vorstehenden Ansprüche,
**dadurch gekennzeichnet, dass** die Datenverarbeitungseinheit (18) dazu vorgesehen und angepasst ist, um Daten zu erfassen und in die vorbestimmte Anzahl an Halterungssystemen (7) und/oder in das von der vorbestimmten Anzahl an Halterungssystemen (7) gehaltene Sterilgut (2) zu schreiben.

8. Modulares Erfassungssystem gemäß einem der vorstehenden Ansprüche,
**dadurch gekennzeichnet, dass** das modulare Erfassungssystem dazu vorgesehen und ausgebildet ist, um mit zumindest einem weiteren modularen Erfassungssystem zu kommunizieren und Daten auszutauschen.

9. Modulares Erfassungssystem gemäß einem der vorstehenden Ansprüche,
**dadurch gekennzeichnet, dass** die zentrale Elektronikeinheit (5) dazu vorgesehen und ausgebildet ist, um über das zumindest eine Verteilermodul (10) das zumindest eine jeweilige Antennensystem (8) der vorzugsweise festgelegten Anzahl an Halterungssystemen (7) auszulesen und die erhaltenen Daten und/ oder Informationen an eine externe Einheit (12), vorzugsweise ein Smartphone und/oder eine App und/ oder ein Steuergerät, zu übertragen, welche dazu vorgesehen und ausgebildet ist, die erhaltenen Daten und/ oder Informationen, vorzugsweise Artikelnummer, Seriennummer, Antennenplatz, Lageplatz und/ oder Informationen bezüglich Aufbereitungszyklen, visuell auszugeben.

10. Halterungssystem (7) zum Halten von Sterilgut (2), insbesondere von chirurgischen Instrumenten und/ oder Motorensystemen und/ oder Medizinprodukten, **dadurch gekennzeichnet, dass** das Halterungssystem (7) ein Sensorsystem (11) zum eigenständigen Erfassen von Prozessschritten aufweist, wobei das Halterungssystem (7) dazu vorgesehen und ausgebildet ist, an ein modulares Erfassungssystem gemäß einem der vorstehenden Ansprüche angeschlossen zu werden.

11. Siebkorbsystem zum Überwachen und Rückverfolgen von Sterilgut (2), insbesondere von chirurgischen Instrumenten und/ oder Motorensystemen und/ oder Medizinprodukten, in einem Sterilgutkreislauf, **dadurch gekennzeichnet, dass** ein erster und zumindest ein weiterer Siebkorb (1) gestapelt/ stapelbar vorgesehen und ausgebildet sind, in welche jeweils ein modulares Erfassungssystem gemäß einem der vorstehenden Ansprüche 1 bis 7 eingelegt ist, wobei
nur das modulare Erfassungssystem des ersten Siebkorbs (1) die zentrale Elektronikeinheit (5) aufweist, welche mit dem modularen Erfassungssystem des zumindest einen weiteren Siebkorbs (1) ohne dem Modul der zentralen Elektronikeinheit (5), vorzugsweise über weitere Verteilermodule (10), verbunden/ verbindbar ist.

12. Verfahren zur Überwachung eines Sterilgutkreislaufs mit zumindest einem modularen Erfassungssystem gemäß einem der vorstehenden Ansprüche 1 bis 9, mit den folgenden Schritten:
- Erfassen und Zwischenspeichern von Prozessschritten und/ oder Sensorwerten von zumindest einem Halterungssystem (7);
- Anschließen/ Verbinden des zumindest einen Halterungssystems 7() mit der zentralen Elektronikeinheit (5) des modularen Erfassungssystems über zumindest eine Daten- und Antennenschnittstelle;
- Übertragen von Daten, insbesondere der Prozessschritte und der Sensorwerte, des zumindest einen Halterungssystems (7) an die zentrale Elektronikeinheit (5), insbesondere dessen Datenverarbeitungseinheit (18), des modularen Erfassungssystems;
- Aufladen der Energiespeichereinheit;
- Auswerten und Evaluieren der Daten in der Datenverarbeitungseinheit (18) des modularen Erfassungssystems;
- Auslesen einer Instrumenten-ID zum Abgleichen mit einer hinterlegten Liste für das zumindest eine angeschlossene/ verbundene Halterungssystem (7); und
- Informationsausgabe bei einem negativen Abgleichergebnis.

## Claims

1. A modular detection system for insertion into a sieve basket (1) for detecting, monitoring and tracing sterile goods (2), in particular surgical instruments and/or motor systems and/or medical products in a sterilization cycle, wherein the modular detection system is provided and configured as a sieve-basket insert (3) which can be inserted into the sieve basket (1) directly or arranged on a base plate (4), with the following modules which can be individually and optionally electrically coupled
a central electronic unit (5), which is preferably configured with at least one sensor, an energy storage unit (17), a data processing unit (18), a communication unit (19), and a writing and reading unit (20), preferably via NFC,
a defined or freely selectable number of slots (6) for connecting and/or coupling a predetermined number of retainer systems (7), which are provided and configured for retaining sterile goods (2) and each with at least one antenna system (8) for reading a transponder, preferably an NFC transponder, of the sterile goods (2), and
a preferably fixed number of conducting tracks (9), in particular data lines and antenna lines and/or connections, which are provided and configured to electrically connect the slots (6) and/or retainer systems (7) to each other and/or to the central electronic unit (5).

2. The modular detection system according to claim 1, **characterized in that** in the central electronic unit (5), at least one distribution module (10) is provided and configured to drive at least two retainer systems (7) via the respective antenna system (8).

3. The modular detection system according to one of the preceding claims, **characterized in that** multiple distribution modules (10) are provided and configured in such a way that they are connected/connectable in a series with the central electronic unit (5) in order to drive the respective distribution modules (10) in series and thus to switch through the at least one respective antenna system (8) in order to read out the sterile goods (2) lying on the at least one respective antenna system (8).

4. The modular detection system according to one of the preceding claims, **characterized in that** the at least one respective antenna system (8) of the number of retainer systems (7) is provided and configured as an NFC antenna.

5. The modular detection system according to one of the preceding claims, **characterized in that** the number of retainer systems (7) is each provided and configured with a sensor system (11) for detecting and temporarily storing data, wherein the sensor system (11) has at least one sensor, a data processing unit, a temporary memory, and a rechargeable energy storage unit.

6. The modular detection system according to one of the preceding claims, **characterized in that** the central electronic unit (5) is provided and configured for detecting and temporarily storing data and is in communication with the sensor system (11) of the predetermined number of retainer systems (7) and/or with the sterile goods (2) held in the predetermined number of retainer systems (7).

7. The modular detection system according to one of the preceding claims, **characterized in that** the data processing unit (18) is provided and adapted to detect and write data to the predetermined number of retainer systems (7) and/or to the sterile goods (2) held by the predetermined number of retainer systems (7).

8. The modular detection system according to one of the preceding claims, **characterized in that** the modular detection system is provided and adapted to communicate and exchange data with at least one further modular detection system.

9. The modular detection system according to one of the preceding claims, **characterized in that** the central electronic unit (5) is provided and configured to read out, via the at least one distribution module (10), the at least one respective antenna system (8) of the preferably fixed number of retainer systems (7) and to transmit the received data and/or information to an external unit (12), preferably a smartphone and/or an app and/or a control device, which is provided and configured to visually output the received data and/or information, preferably item number, serial number, antenna location, position and/or information regarding processing cycles.

10. A retainer system (7) for retaining sterile goods (2), in particular surgical instruments and/or motor systems and/or medical products, **characterized in that** the retainer system (7) comprises a sensor system (11) for autonomously detecting of process steps, wherein the retainer system (7) is provided and configured to be connected to a modular detection system according to one of the preceding claims.

11. A sieve basket system for monitoring and tracing sterile goods (2), in particular surgical instruments and/or motor systems and/or medical products, in a sterile goods cycle, **characterized in that** a first and at least one further sieve basket (1) are provided and configured in a stacked/stackable manner, into each of which a modular detection system according to one of the preceding claims 1 to 7 is inserted, wherein
only the modular detection system of the first sieve basket (1) comprises the central electronic unit (5), which is connected/connectable to the modular detection system of the at least one further sieve basket (1) without the module of the central electronic unit (5), preferably via further distribution modules (10).

12. A method for monitoring a sterile goods cycle with at least one modular detection system according to one of the preceding claims 1 to 9, comprising the following steps:
- detecting and temporarily storing process steps and/or sensor values of at least one retainer system (7);
- connecting/coupling the at least one retainer system (7) to the central electronic unit (5) of the modular detection system via at least one data and antenna interface;
- transmitting data, in particular the process steps and the sensor values, of the at least one retainer system (7) to the central electronic unit (5), in particular its data processing unit (18), of the modular detection system;
- charging the energy storage unit;
- analyzing and evaluating the data in the data processing unit (18) of the modular detection system;
- reading out an instrument ID for matching with a stored list for the at least one connected/coupled retainer system (7); and
- outputting information in case of a negative matching result.

## Revendications

1. Système de détection modulaire destiné à être inséré dans un panier à tamis (1) pour la détection, la surveillance et le traçage de produits stériles (2), en particulier d'instruments chirurgicaux et/ou de systèmes motorisés et/ou de produits médicaux dans un circuit de stérilisation, le système de détection modulaire étant prévu et réalisé sous la forme d'un insert de panier à tamis (3), lequel peut être inséré directement dans le panier à tamis (1) ou disposé sur une plaque de base (4) dans celui-ci, comportant des modules suivants pouvant être couplés individuellement et éventuellement électriquement :
une unité électronique centrale (5), qui est de préférence réalisée avec au moins un capteur, une unité d'accumulation d'énergie (17), une unité de traitement de données (18), une unité de communication (19) et une unité d'écriture et de lecture (20), de préférence par NFC,
un nombre défini ou librement sélectionnable de postes d'enfichage (6) pour le raccordement et/ou la liaison d'un nombre prédéfini de systèmes de support (7) destinés à supporter un produit stérile (2), lesquels systèmes de support sont prévus et réalisés avec respectivement au moins un système d'antenne (8) pour la lecture d'un transpondeur, de préférence un transpondeur NFC, du produit stérile (2), et
un nombre de préférence fixe de pistes conductrices (9), en particulier de lignes et/ou de connexions de données et d'antennes, lesquelles sont prévues et réalisées de façon à connecter électriquement les postes d'enfichage (6) et/ou les systèmes de support (7) entre eux et/ou à l'unité électronique centrale (5).

2. Système de détection modulaire selon la revendication 1,
**caractérisé en ce qu'**au moins un module de distribution (10) est prévu et réalisé dans l'unité électronique centrale (5), lequel module de distribution est configuré pour commander respectivement au moins deux systèmes de support (7) par l'intermédiaire du système d'antenne (8) respectif.

3. Système de détection modulaire selon l'une des revendications précédentes, **caractérisé en ce que** plusieurs modules de distribution (10) sont prévus et réalisés de façon à être connectés/pouvoir être connectés, dans un montage en série, à l'unité électronique centrale (5), pour commander successivement les modules de distribution (10) respectifs et connecter ainsi l'au moins un système d'antenne (8) respectif pour lire le produit stérile (2) situé sur l'au moins un système d'antenne (8) respectif.

4. Système de détection modulaire selon l'une des revendications précédentes, **caractérisé en ce que** l'au moins un système d'antenne (8) respectif du nombre de systèmes de support (7) est prévu et réalisé sous la forme d'une antenne NFC.

5. Système de détection modulaire selon l'une des revendications précédentes, **caractérisé en ce que** le nombre de systèmes de support (7) est prévu et réalisé respectivement avec un système de capteurs (11) pour la détection et le stockage intermédiaire de données, le système de capteurs (11) ayant au moins un capteur, une unité de traitement de données, une mémoire temporaire et une unité d'accumulation d'énergie rechargeable.

6. Système de détection modulaire selon l'une des revendications précédentes, **caractérisé en ce que** l'unité électronique centrale (5) est prévue et réalisée pour la détection et le stockage intermédiaire de données et pour être en connexion de communication avec le système de capteurs (11) du nombre prédéfini de systèmes de support (7) et/ou avec le produit stérile (2) supporté dans le nombre prédéfini de systèmes de support (7).

7. Système de détection modulaire selon l'une des revendications précédentes, **caractérisé en ce que** l'unité de traitement de données (18) est prévue et adaptée pour détecter des données et pour les écrire dans le nombre prédéfini de systèmes de support (7) et/ou dans le produit stérile (2) supporté dans le nombre prédéfini de systèmes de support (7).

8. Système de détection modulaire selon l'une des revendications précédentes, **caractérisé en ce que** le système de détection modulaire est prévu et réalisé pour communiquer et échanger des données avec au moins un autre système de détection modulaire.

9. Système de détection modulaire selon l'une des revendications précédentes, **caractérisé en ce que** l'unité électronique centrale (5) est prévue et réalisée pour lire, par l'intermédiaire de l'au moins un module de distribution (10), l'au moins un système d'antenne (8) respectif du nombre de préférence fixe de systèmes de support (7) et pour transmettre les données et/ou informations obtenues à une unité externe (12), de préférence un téléphone intelligent et/ou une application et/ou un appareil de commande, laquelle est prévue et réalisée pour émettre visuellement les données et/ou informations obtenues, de préférence le numéro de référence, le numéro de série, l'emplacement d'antenne, l'emplacement et/ou des informations concernant les cycles de traitement.

10. Système de support (7) destiné à supporter des produits stériles (2), en particulier des instruments chirurgicaux et/ou des systèmes motorisés et/ou des produits médicaux, **caractérisé en ce que** le système de support (7) présente un système de capteurs (11) pour la détection autonome d'étapes de processus, le système de support (7) étant prévu et réalisé pour être raccordé à un système de détection modulaire selon l'une des revendications précédentes.

11. Système de panier à tamis pour la surveillance et le traçage de produits stériles (2), en particulier d'instruments chirurgicaux et/ou de systèmes motorisés et/ou de produits médicaux, dans un circuit de produits stériles,
**caractérisé en ce qu'**un premier et au moins un autre panier à tamis (1) sont prévus et réalisés empilés/empilables, respectivement un système de détection modulaire selon l'une des revendications précédentes 1 à 7 étant inséré dans ledit panier à tamis, seul le système de détection modulaire du premier panier à tamis (1) présentant l'unité électronique centrale (5), laquelle est connectée/peut être connectée au système de détection modulaire de l'au moins un autre panier à tamis (1) ne comprenant pas le module de l'unité électronique centrale (5), de préférence par l'intermédiaire d'autres modules de distribution (10).

12. Procédé pour la surveillance d'un circuit de produits stériles, comportant au moins un système de détection modulaire selon l'une des revendications précédentes 1 à 9, comportant les étapes suivantes :
- détection et stockage intermédiaire d'étapes de processus et/ou de valeurs de capteur d'au moins un système de support (7) ;
- raccordement/liaison de l'au moins un système de support (7) à l'unité électronique centrale (5) du système de détection modulaire par l'intermédiaire d'au moins une interface de données et d'antenne ;
- transmission de données, en particulier des étapes de processus et des valeurs de capteur, de l'au moins un système de support (7) à l'unité électronique centrale (5), en particulier à son unité de traitement de données (18), du système de détection modulaire ;
- chargement de l'unité d'accumulation d'énergie ;
- analyse et évaluation des données dans l'unité de traitement de données (18) du système de détection modulaire ;
- lecture d'un ID d'instrument pour la comparaison avec une liste enregistrée pour l'au moins un système de support (7) raccordé/relié ;
- sortie d'informations en cas de résultat de comparaison négatif.
